# EUROPÄISCHE PATENTANMELDUNG

(11) **EP 3 915 627 A2**
(43) Veröffentlichungstag der Anmeldung: **01.12.2021**
(21) Anmeldenummer: 21174254.9
(22) Anmeldetag: 18.05.2021
(51) Int. Cl.: A61M 25/02, A61M 5/158

(54) **TRAGBARE HALTERUNG FÜR EXTRACORPURALE ABSCHNITTE MEDIZINISCHER KATHETERSCHLÄUCHE SOWIE DEREN VERWENDUNG**

(30) Priorität: 20.05.2020 DE 202020102898 U
(71) Anmelder: Wienholz, Viviane, 01279 Dresden (DE)
(72) Erfinder: Wienholz, Viviane, 01279 Dresden (DE)
(74) Vertreter: Rauschenbach, Marion

(57) **Zusammenfassung**

Die vorliegende Anmeldung bezieht sich auf das Gebiet der Medizin sowie medizinischer Hilfsmittel und betrifft eine tragbare Halterung für extracorpurale Abschnitte medizinischer Katheterschläuche sowie deren Verwendung.

Die Aufgabe besteht in der Angabe einer Halterung für extracorpurale Abschnitte medizinischer Katheterschläuche, die ein Verdrehen, Verwinden und oder Brechen der extracorpuralen Abschnitte verhindert, die Bewegungsfreiheit des Patienten während des Tragens nicht beeinträchtigt und ein sicheres und einfaches Anordnen und Entfernen der Halterung vom menschlichen Körper ermöglicht.

Gelöst wird die Aufgabe durch eine tragbare Halterung für extracorpurale Abschnitte medizinischer Katheterschläuche, die eine aus mehreren Abschnitten bestehende und wiederverschließbare Tasche aufweist, wobei an ihrer Rückseite ein oder mehrere Befestigungsmittel zum gürtel- oder bandfreien Anordnen der Tasche am menschlichen Körper vorhanden sind und mindestens ein Abschnitt der Tasche mindestens ein Fixierelement (4) mit mindestens einer kanalartig ausgebildeten Vertiefung (5) aufweist.

## Beschreibung

Die vorliegende Erfindung bezieht sich auf das Gebiet der Medizin sowie medizinischer Hilfsmittel und betrifft eine tragbare Halterung für extracorpurale Abschnitte medizinischer Katheterschläuche sowie deren Verwendung. Die erfindungsgemäße tragbare Halterung kann beispielsweise für PEG-Katheter, PEJ-Katheter und/oder Dialyse-Katheter, aber auch für medizinische Drainageschläuche zur Wundbehandlung, eingesetzt werden.

Aus der Medizin ist in Verbindung mit Kathetern und Sonden unter anderem die perkutane endoskopische Gastrostomie und die perkutane endoskopische Jejunostomie bekannt, bei denen über eine Sonde durch die Bauchdecke hindurch Nahrung in den oberen Dünndarm verabreicht wird beziehungsweise ein endoskopisch angelegter künstlicher Zugang von außen durch die Bauchdecke in den Magen gelegt wird. Durch diese Zugänge kann ein elastischer Kunststoffschlauch gelegt werden.

Diese beispielsweise enteralen Ernährungs- und Versorgungssonden sind Katheterschläuche, die aus der Bauchdecke des Körpers herausgeführt sind und einen Sondenport für das Zuführen von Nahrung oder das Ausscheiden und Entfernen überflüssiger Stoffwechselprodukte aufweisen.

Aus dem Stand der Technik sind Halterung für Katheterschläuche bekannt, die insbesondere darauf gerichtet sind, im direkten Austrittsbereich des Katheters aus der Bauchdecke eines Patienten eine sterile Wundversorgung zu gewährleisten.

Aus der DE 23 62 947 ist eine Halterung für Katheter bekannt, bei der das in eine Fistel des Körpers eingeführte Katheterrohr mittels eines im Bereich der äußeren Fistelmündung angelegten Gürtels an der aus der Körperoberfläche herausragenden Stelle des Katheters gehalten wird. Dabei wird der Katheter durch ein in einer Lasche angeordnetes Katheterloch mit mindestens zwei radial vom Lochrand nach außen verlaufenden Einschnitten hindurchgeführt.

Aus der EP 409 583 A1 ist eine Haltevorrichtung insbesondere für Katheter bekannt, mit einem ersten Kissen aus für medizinische Zwecke geeignetem Klebstoff, dessen Oberfläche durch eine ablösbare Schutzschicht abgedeckt ist und dessen zweite Oberfläche durch eine Kunststofffolie bedeckt ist, und mit einem an der Folie befestigten zweiten Kissen aus für medizinische Zwecke geeignetem Klebstoff, dessen von dem ersten Kissen wegweisende Oberfläche durch eine ablösbare Schutzschicht und dessen andere Oberfläche durch eine Kunststofffolie abgedeckt ist, wobei die beiden Kunststofffolien über einen Mittelabschnitt miteinander befestigt sind, dessen Fläche wesentlich kleiner ist als die Flächen sowohl des ersten als auch des zweiten Kissens. Das zweite Kissen ist kreisförmig oder nahezu kreisförmig oder oval ausgebildet, wobei bei nahezu Kreisform oder Ovalform die Länge der Nebenachse mindestens 75% der Länge der Hauptachse beträgt.

Aus der DE 20 2011 101 529 U1 ist eine Sterilschutzanordnung bekannt, aufweisend einen Katheter mit mindestens einem Sondenlumen und zugehörigem Sondenport zum Einschieben einer Sonde in das Sondenlumen, eine transparente Folie, welche auf eine Fläche von mindestens 100 Quadratzentimetern ausbreitbar ist, im Bereich des Sondenports auf dem Katheter aufliegt und dazu angepasst ist, die Durchführung einer Sonde durch die Folie zu ermöglichen, wobei zumindest die dem Katheter zugewandte Seite der Folie steril ist.

Auch bekannt sind abdominale Ernährungsschlauchgürtel, in die extracorpurale Abschnitte von Kathetern eingeführt und so am Körper gehalten werden.

Nachteilig bei den bekannten Lösungen ist, dass die extracorpuralen Abschnitte medizinischer Katheterschläuche durch die Bewegung des Patienten sich verdrehen, verwinden und brechen können und dadurch in ihrer Funktion beeinträchtigt sind. Auch ist nachteilig, dass das Fixieren der Katheterschläuche im direkten Austrittsbereich des Körpers zum schnellen Ein- und Verwachsen mit der Körperhaut führt. Weiterhin ist nachteilig, dass die extracorpuralen Abschnitte der Katheterschläuche sowie bekannte Ernährungsschlauchgürtel die Bewegungsfreiheit des Patienten massiv einschränken und behindern.

Die Aufgabe der vorliegenden Erfindung besteht darin, eine Halterung für extracorpurale Abschnitte medizinischer Katheterschläuche bereitzustellen, mit der die vorgenannten Nachteile des Standes der Technik beseitigt werden.

Die Aufgabe wird durch die in den Patentansprüchen angegebene Erfindung gelöst. Vorteilhafte Ausgestaltungen sind Gegenstand der Unteransprüche, wobei die Erfindung auch Kombinationen der einzelnen Patentansprüche im Sinne einer UND-Verknüpfung einschließt, solange sie sich nicht gegenseitig ausschließen.

Gelöst wird die erfindungsgemäße Aufgabe durch eine tragbare Halterung für extracorpurale Abschnitte medizinischer Katheterschläuche, die eine aus mehreren Abschnitten bestehende und wiederverschließbare Tasche mit Mitteln zur seitlichen Zuführung, Aufnahme und Halterung der Katheterschläuche aufweist, wobei die Tasche an ihrer Rückseite ein oder mehrere Befestigungsmittel zum gürtel- oder bandfreien Anordnen der Tasche am menschlichen Körper aufweist, und wobei mindestens ein Abschnitt der Tasche mindestens ein Fixierelement mit mindestens einer kanalartig ausgebildeten Vertiefung aufweist, in der der extracorpurale Katheterabschnitt mindestens in seiner Lage, Länge und/oder Anordnung fixierbar ist.

Vorteilhafterweise ist das Fixierelement eine hergestellte Platte ist, die aus einem elastisch verformbaren Polymermaterial hergestellt, wobei besonders vorteilhaft das Fixierelement aus PE oder PET hergestellt ist.

Auch ist es vorteilhaft, wenn die kanalartig ausgebildete Vertiefung ein mäanderförmiger Kanal und/oder bogenförmige Kanalabschnitte sind.

Zudem ist vorteilhaft, wenn das Fixierelement mindestens ein wiederverschließbares Sicherungselement aufweist.

In einer vorteilhaften Ausgestaltung der Halterung sind an der Rückseite der Tasche mindestens teilweise adhäsive Mittel zum Herstellen einer stoffschlüssigen Verbindung mit dem menschlichen Körper vorhanden und besonders vorteilhaft das adhäsive Mittel ein hautverträglicher und/oder atmungsaktiver Klebstoff und/oder eine hautverträgliche und/oder atmungsaktive Folie.

Vorteilhafterweise ist das Befestigungsmittel als zweiteiliges Verbindungselement ausgebildet, wobei ein erstes Verbindungselement an der Rückseite der Tasche angeordnet ist und ein zweites Verbindungselement für die Anordnung am menschlichen Körper vorgesehen ist, wobei das erste und zweite Verbindungselementteil form- und/oder kraftschlüssig miteinander verbindbar ist. Besonders vorteilhaft ist es dabei, wenn das zweiteilige Verbindungselement als Rastring oder Klickverbindung ausgebildet ist und ganz beonders vorteilhaft ist der Rastring oder die Klickverbindung oval, dreieckig, quadratisch und/oder rechteckig ausgebildet.

Weiterhin ist vorteilhaft, wenn mindestens die Tasche aus einem hautfreundlichen, atmungsaktiven, waschbaren und/oder feuchtigkeitsaufnehmenden Material besteht.

Auch vorteilhafterweise sind mindestens zwei Abschnitte der Tasche mittels Verschlusselementen und ganz besonders vorteilhaft mittels Klettbänder, Knöpfe und/oder Reißverschlüsse, wiederverschließbar.

In einer vorteilhaften Ausgestaltung der Halterung ist die Tasche im Innen- und/oder Außenbereich wasserdicht und wasserabweisend ausgebildet.

Die erfindungsgemäße Halterung kann zur Halterung mindestens eines extracorpuralen Abschnittes von Katheterschläuchen für PEG-Katheter, PEJ-Katheter und/oder Dialyse-Katheter verwendet werden.

Mit der erfindungsgemäßen Lösung wird eine tragbare Halterung für extracorpurale Abschnitte medizinischer Katheterschläuche bereitgestellt, mit der ein sicheres, geschütztes und komfortables Tragen der aus dem menschlichen Körper heraustretenden Katheter- oder auch Drainageschläuche ohne wesentliche Einschränkung der Bewegungsfreiheit ermöglicht wird. Die vorgeschlagene Halterung ist insbesondere einfach in der Handhabung und Funktion, lässt sich in einfacher Weise am menschlichen Körper befestigen und wieder lösen und verhindert insbesondere eine Funktionsstörung oder Beschädigung der extracorpuralen Abschnitte der Katheter -oder Drainageschläuche.

Erreicht werden die vorgenannten Vorteile mit einer Halterung, die eine aus mehreren Abschnitten bestehende und wiederverschließbare Tasche aufweist, die Mittel zur seitlichen Zuführung, Aufnahme und Halterung der Katheterschläuche bereitstellt, wobei an deren Rückseite ein oder mehrere Befestigungsmittel zum gürtel- oder bandfreien Anordnen der Tasche am menschlichen Körper vorhanden sind. Ein gürtel-oder bandfreies Anordnen der Tasche am menschlichen Körper hat den Vorteil, dass Patienten ohne wesentliche Einschränkungen den gewünschten und/oder notwendigen Aktivitäten nachgehen können, ohne dass dabei die Position und der Sitz der Halterung am menschlichen Körper verändert wird.

In einer vorteilhaften Ausgestaltung ist das Befestigungsmittel als zweiteiliges Verbindungselement ausgebildet, wobei ein erstes Verbindungselement im Bereich eines mittleren Abschnittes und an der Rückseite der Tasche angeordnet ist und ein zweites Verbindungselement für die Anordnung am menschlichen Körper vorgesehen ist. Das erste und zweite Verbindungselement ist form- und/oder kraftschlüssig miteinander verbindbar.

Mit einer zweiteiligen Ausbildung des Verbindungselementes wird erreicht, dass das erste Verbindungselement beispielsweise dauerhaft an der Rückseite der Tasche verbleiben kann, während das zweite Verbindungselement ein austauschbares Verbindungselement ist, dass am menschlichen Körper zeitweise verbleiben und bei Bedarf und/oder aus hygienischen Gründen ausgetauscht werden kann. Besonders vorteilhaft ist das zweiteilige Verbindungselement als Rastring oder Klickverbindung ausgebildet, wodurch ein schnelles sowie sicheres Verbinden und Lösen der Tasche vom menschlichen Körper ermöglicht wird.

Um das Verdrehen oder Rotieren der Halterung beim Tragen zu beseitigen, kann besonders vorteilhaft vorgesehen sein, dass der Rastring oder die Klickverbindung oval, dreieckig, quadratisch und/oder rechteckig ausgebildet ist.

Erfindungsgemäß ist vorgesehen, dass mindestens ein Abschnitt der Tasche mindestens ein Fixierelement mit mindestens einer kanalartig ausgebildeten Vertiefung aufweist, in der der extracorpurale Katheterabschnitt mindestens in seiner Lage, Länge und/oder Anordnung fixierbar ist.

Die kanalartig ausgebildete Vertiefung kann ein mäanderförmiger Kanal oder können bogenförmige Kanalabschnitte sein. Ein mäanderförmiger Kanal oder bogenförmige Kanalabschnitte als kanalartige Vertiefungen verhindern insbesondere das Knicken des Katheterschlauches und reduziert zudem die Gefahr des Herausziehens des Katheterschlauches innerhalb der Tasche bei Aktivitäten des Patienten.

Die kanalartig ausgebildete Vertiefung ermöglicht das definierte Einlegen, Positionieren und Fixieren des extracorpuralen Katheterabschnittes, sodass der in der Regel aus einem Kunststoff bestehende Katheter- oder Drainageschlauch in seiner Lage gesichert wird. Dadurch wird insbesondere die Funktion des Katheters auch innerhalb der Halterung gewährleistet und dessen Haltbarkeit wesentlich verlängert, wodurch Kosten gespart und die Handhabung erleichtert werden.

In einer vorteilhaften Ausgestaltung des Fixierelementes kann vorgesehen sein, dass zusätzlich mindestens ein wiederverschließbares Sicherungselement zur zusätzlichen Fixieren des Kateheterschlauches vorgesehen ist, dass besonders vorteilhaft eine an die Form eines Katheter- oder Drainageschlauches angepasste Innenwölbung aufweist. Mit einem derartigen Sicherungselement wird insbesondere eine konstante Länge des extracorpuralen Katheterabschnittes innerhalb der Tasche gewährleistet. Zudem wird bei einer auftretenden Zugbelastung des extracorpuralen Katheter- oder Drainageabschnittes ein unbeabsichtigtes Herausziehen aus der Halterung und/oder ein Öffnen des Katheterschlauchverschlusses wirksam vermieden.

Zum Verbessern des Tragekomforts sowie zum Vermeiden von Stauchungen kann vorgesehen sein, dass das Fixierelement aus einem elastisch verformbaren Polymermaterial hergestellt ist. Besonders vorteilhaft ist, wenn das Sicherungselement aus Polyethylen (PE) oder Polyethylenterephthalat (PET) hergestellt ist. Die vorgenannten Materialien weisen insbesondere eine hohe Flexibilität und gute Festigkeit auf. Zudem sind PE und PET Kunststoffmaterialien, die bei höheren Temperaturen, wie sie beispielsweise bei Waschgängen zur Reinigung der Halterung auftreten können, eine gute Temperatur- und Formbeständigkeit aufweisen. Der Einsatz eines elastisch verformbaren Polymermaterials ermöglicht insbesondere eine verbesserte Bewegungsfreiheit des Patienten, da sich das Material bei der Bewegung an die sich verändernde Körperform anpasst und gleichzeitig eine sichere Fixierung des Katheter- oder Drainageschlauches gewährleistet.

Für eine sichere Anordnung der Halterung am menschlichen Körper kann in einer weiteren vorteilhaften Ausgestaltung der Erfindung vorgesehen sein, dass ein dem Körper zugewandter rückseitiger Bereich der Tasche mindestens teilweise adhäsive Mittel zur zusätzlichen Positionssicherung der Halterung am menschlichen Körper aufweist. Die zusätzlichen adhäsiven Mittel verhindern, dass die Tasche bei Bewegung des Patienten verrutscht und/oder knickt und dadurch der in der Tasche angeordnete Katheterschlauch aus dem Körper gezogen werden. Derartige adhäsive Mittel können beispielsweise ein hautverträglicher und atmungsaktiver Klebstoff und/oder eine ein- oder mehrmalig verwendbare hautverträgliche und/oder atmungsaktive Folie sein.

In einer vorteilhaften Ausgestaltung der Halterung kann mindestens die Tasche aus einem hautfreundlichen, atmungsaktiven, waschbaren und/oder feuchtigkeitsaufnehmenden Material bestehen.

Zur Nutzung der Halterung auch im Wasser kann vorteilhafterweise vorgesehen sein, dass die Tasche wasserdicht und wasserabweisend ausgebildet ist. So ist vorstellbar, dass die Tasche im Innen- und/oder Außenbereich eine wasserdichte Beschichtung aufweist. Die Beschichtung kann beispielsweise aus Neopren hergestellt sein.

Zudem kann der Verschluss der Tasche mittels wasserdichter Verschlusselemente, beispielsweise eines wasserdichten und umlaufenden Reißverschlusses, ausgebildet sind, die einerseits die seitliche Zuführung extracorpuraler Katheterabschnitte ermöglicht und gleichzeitig das Eindringen von Wasser in das Innere der geschlossenen Tasche verhindert.

Zusammenfassend werden mit der erfindungsgemäß vorgeschlagenen Halterung für extracorpurale Abschnitte medizinischer Katheter- oder Drainageschläuche mehrere technische Vorteile und Wirkungen erreicht, nämlich
- das Schützen der Katheterabschnitte vor Verschmutzung und ungewolltem Öffnen des Katheterverschlusses,
- die Sicherstellung der Funktion der Katheterabschnitte während des Tragens der Halterung,
- die einfache und komfortable Fixierung der Katheter- oder Drainageabschnitte innerhalb einer Tasche mit Vermeidung von Zugbeanspruchung und Beschädigung der Katheter- oder Drainageabschnitte,
- das einfache und kostengünstige Anbringen und Entfernen der Halterung an den oder vom menschlichen Körper,
- der Entfall von Bändern oder Gürteln, wodurch eine verbesserte Bewegungsfreiheit und ein positionssicherer Halt ermöglicht werden.

### Ausführungsbeispiel

Nachfolgend wird die Erfindung anhand eines Ausführungsbeispiels näher erläutert. Die dazugehörigen Figuren zeigen
- Figur 1: eine schematische Darstellung der erfindungsgemäßen Halterung im geöffneten Zustand mit Sicherungselement, und
- Figur 2: eine schematische Darstellung der erfindungsgemäßen Halterung mit rückseitigem Befestigungsmittel.

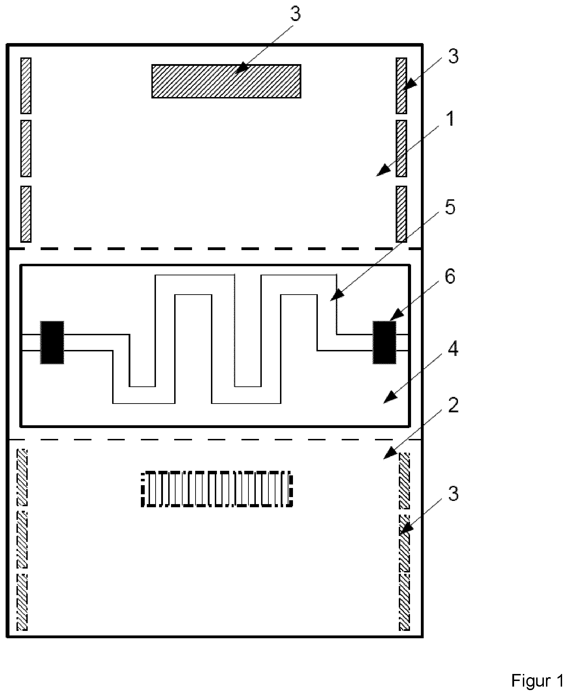

Figur 1 zeigt in schematischer Darstellung die tragbare Halterung für einen extracorpuralen Abschnitt eines Katheterschlauches mit einer geöffnet dargestellten Tasche. Im oberen Abschnitt 1 der Tasche sind innenseitig mehrere Verschlusselemente 3 als Flauschteile und im unteren Bereich 2 der Tasche außenseitig die zugehörigen Hakenteile eines Klettverschlusses angeordnet, die zum sicheren Verschließen der Tasche vorgesehen sind.

Der mittlere Abschnitt der Tasche weist ein Fixierelement 4 aus Polyethylen auf, dass mit der Tasche stoffschlüssig verbunden und als Platte ausgebildet ist. Der extracorpurale Abschnitt des Katheterschlauches ist seitlich in die Tasche linksseitig dem Fixierelement 4 zuführbar und in die mäanderförmig und kanalartig ausgebildete Vertiefung 5 einlegbar, wodurch dessen Lage und Länge fixiert ist. Zur Sicherung des Abschnittes des Katheterschlauches sind links und rechts des Fixierelementes 4 zwei Sicherungselemente 6 angeordnet, die als Schnappverschlüsse mit an die Form des Katheterschlauches angepasster Innenwölbung ausgebildet sind. Die Innenwölbung ist zusätzlich mit einer Gummierung versehen, die eine verbesserte Reibung und damit Fixierung des Katheterschlauches ermöglicht.

Die Tasche besteht aus einem hautfreundlichen und waschbaren Baumwollstoff. Nach der Befestigung des Katheterschlauches kann die Tasche an den horizontal unterbrochen dargestellten Linien derart zusammengeklappt werden, dass der untere Abschnitt 2 zuerst und nachfolgend der obere Abschnitt 1 der Tasche umgeklappt werden und der Verschluss der Tasche über die Klettverschlüsse 3 realisiert wird.

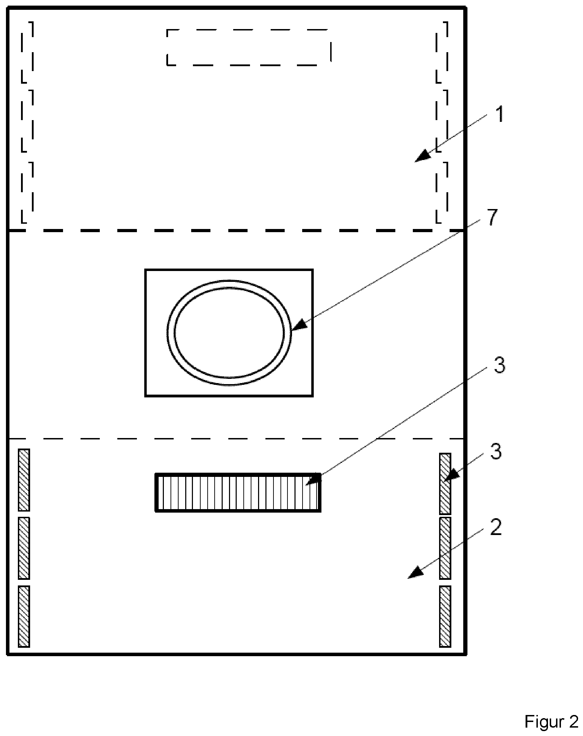

Zum Befestigen und Lösen der Halterung am und vom menschlichen Körper eines Patienten ist gemäß Figur 2 auf der Rückseite der Tasche im Bereich des mittleren Abschnittes der Tasche ein zweiteiliges Befestigungsmittel 7 aus PET in Form eines ersten oval ausgebildeten Schnappverschlusselementes vorgesehen, dass mit einem zweiten Schnappverschlusselementes (nicht dargestellt), das am menschlichen Körper mittels einer hautfreundlichen und atmungsaktiven Klebefolie angeordnet ist, ein formschlüssiges Verbinden des ersten und zweiten Schnappverschlusselementes und damit ein sicheres Befestigen und einfaches Lösen der Halterung am und vom menschlichen Körper ermöglicht.

### Bezugszeichenliste

1 oberer Abschnitt der Tasche
2 unterer Abschnitt der Tasche
3 Verschlusselemente
4 Fixierelement
5 Kanalartige Vertiefung
6 Sicherungselement
7 Befestigungsmittel

## Patentansprüche

1. Tragbare Halterung für extracorpurale Abschnitte medizinischer Katheterschläuche, die eine aus mehreren Abschnitten bestehende und wiederverschließbare Tasche mit Mitteln zur seitlichen Zuführung, Aufnahme und Halterung der Katheterschläuche aufweist, wobei die Tasche an ihrer Rückseite ein oder mehrere Befestigungsmittel zum gürtel- oder bandfreien Anordnen der Tasche am menschlichen Körper aufweist, und wobei mindestens ein Abschnitt der Tasche mindestens ein Fixierelement mit mindestens einer kanalartig ausgebildeten Vertiefung aufweist, in der der extracorpurale Katheterabschnitt mindestens in seiner Lage, Länge und/oder Anordnung fixierbar ist.

2. Halterung nach Anspruch 1, bei der das Fixierelement eine hergestellte Platte ist, die aus einem elastisch verformbaren Polymermaterial hergestellt ist.

3. Halterung nach Anspruch 2, bei das Fixierelement aus PE oder PET hergestellt ist.

4. Halterung nach mindestens einem der vorhergehenden Ansprüche, bei der die kanalartig ausgebildete Vertiefung ein mäanderförmiger Kanal oder bogenförmige Kanalabschnitte sind.

5. Halterung nach mindestens einem der vorhergehenden Ansprüche, bei der das Fixierelement mindestens ein wiederverschließbares Sicherungselement aufweist.

6. Halterung nach mindestens einem der vorhergehenden Ansprüche, bei der an der Rückseite der Tasche mindestens teilweise adhäsive Mittel zum Herstellen einer stoffschlüssigen Verbindung mit dem menschlichen Körper vorhanden sind.

7. Halterung nach Anspruch 6, bei der das adhäsive Mittel ein hautverträglicher und atmungsaktiver Klebstoff und/oder Folie ist.

8. Halterung nach Anspruch 1, bei der das Befestigungsmittel als zweiteiliges Verbindungselement ausgebildet ist, wobei ein erstes Verbindungselement an der Rückseite der Tasche angeordnet ist und ein zweites Verbindungselement für die Anordnung am menschlichen Körper vorgesehen ist, wobei das erste und zweite Verbindungselementteil form- und/oder kraftschlüssig miteinander verbindbar ist.

9. Halterung nach Anspruch 8, bei der das zweiteilige Verbindungselement als Rastring oder Klickverbindung ausgebildet ist.

10. Halterung nach Anspruch 9, bei der der Rastring oder die Klickverbindung oval, dreieckig, quadratisch und/oder rechteckig ausgebildet ist.

11. Halterung nach mindestens einem der vorhergehenden Ansprüche, bei der mindestens die Tasche aus einem hautfreundlichen, atmungsaktiven, waschbaren und/oder feuchtigkeitsaufnehmenden Material besteht.

12. Halterung nach mindestens einem der vorhergehenden Ansprüche, bei der mindestens zwei Abschnitte der Tasche mittels Verschlusselemente, insbesondere mittels Klettbänder, Knöpfe und/oder Reißverschlüsse, wiederverschließbar sind.

13. Halterung nach mindestens einem der vorhergehenden Ansprüche, bei der die Tasche im Innen- und/oder Außenbereich wasserdicht und/oder wasserabweisend ausgebildet ist.

14. Verwendung der Halterung nach mindestens einem der vorhergehenden Ansprüchen 1 bis 13 zur Halterung mindestens eines extracorpuralen Abschnittes von Katheterschläuchen für PEG-Katheter, PEJ-Katheter und/oder Dialyse-Katheter.
